# EUROPÄISCHE PATENTSCHRIFT

(11) **EP 1 498 142 B1**
(45) Veröffentlichungstag und Bekanntmachung des Hinweises auf die Patenterteilung: **21.03.2007**
(21) Anmeldenummer: 02756015.0
(22) Anmeldetag: 10.04.2002
(51) Int. Cl.: A61K 47/32, C08F 220/56, C08F 2/10

(54) **POLYFUNKTIONALES BIOKOMPATIBLES HYDROGEL UND VERFAHREN ZU SEINER HERSTELLUNG**
POLYFUNCTIONAL BIOCOMPATIBLE HYDROGEL AND METHOD FOR THE PRODUCTION THEREOF
HYDROGEL BIOCOMPATIBLE MULTIFONCTIONS ET PROCEDE DE FABRICATION CORRESPONDANT

(43) Veröffentlichungstag der Anmeldung: 19.01.2005
(73) Patentinhaber: Obschestvo S Ogranichennoy Otvetstvennostyu "Vitagel", Moscow, 113149 (RU)
(72) Erfinder: LOPATIN Vladislav Victorovich, Moscow, 119270 (RU)
(74) Vertreter: Jeck, Anton
(86) Internationale Anmeldenummer: PCT/RU2002/000164
(87) Internationale Veröffentlichungsnummer: WO 2003/084573

(56) Entgegenhaltungen:
- WO-A-00/45792
- WO-A-02/16453
- WO-A1-02/16453
- RU-C1- 2 067 873
- RU-C1- 2 122 438
- US-A- 4 769 427
- US-A- 5 338 492
- GARCA O ET AL: "5-Fluorouracil trapping in poly(2-hydroxyethyl methacrylate-co-acrylamide) hydrogels: in vitro drug delivery studies" EUROPEAN POLYMER JOURNAL, PERGAMON PRESS LTD. OXFORD, GB, Bd. 36, Nr. 1, Januar 2000 (2000-01), Seiten 111-122, XP004251519 ISSN: 0014-3057

## Beschreibung

Die Erfindung bezieht sich auf eine Rezeptur und ein Verfahren zur Gewinnung von bioverträglichem Polyacrylamid-Hydrogel, das als Material für medizinische Zwecke, insbesondere als Träger von Menschen- und Tierzellen, die im Organismus von Säugetieren implantiert sind, als Depot für medizinische Präparate während einer längeren, medikamentösen Behandlung, beispielsweise von Geschwülsten oder Abszessen, verwendet werden kann.

Es ist bekannt, dass die Polyacrylamid-Hydrogele (PAAG) genügend billige und in der Herstellung einfache Materialien sind und über eine chemische und biologische Trägheit verfügen. Sie lassen sich leicht mit der gewünschten Dichte und in einer Form synthetisieren, die für die Unterhauteinführung und/oder für die Einführung in Weichteile mit minimalen Verletzungen des Organismus des Patienten geeignet ist.

In der Medizin ist durch die RU 2165263 ein Verfahren zur Behandlung von insulinabhängiger Diabetes bekannt, bei dem heterogene β-Zellen der Bauchspeicheldrüse in das dem Patienten vorher subkutan eingeführte Polyacrylamidgel transplantiert wird, um das sich eine Kapsel gebildet hat.

Durch die RU 2152800 ist auch ein Verfahren zur Kultivierung von heterogenen Zellen von Säugetieren, insbesondere von Leiding-Zellen und von Melanomzellen, durch deren Transplantation in das den Säugetieren vorher eingeführte Polyacrylamidgel bekannt.

Dies eröffnet die Möglichkeit zur Behandlung einer Reihe von Krankheiten durch Transplantation der heterogenen Zellen in den Organismus des Kranken, der die für ihn nötigen Fermente und/oder Hormone erzeugt; dies ermöglicht auch eine Impfbehandlung von onkologischen Krankheiten.

Wie versuchsmäßig festgestellt wurde, hängt die Dauer der Erzeugung der durch die heterogenen Zellen der für den Organismus der Kranken nötigen Zellen bei gleichen Bedingungen von den Eigenschaften des Polyacrylamidgels (PAAG) ab.

Wie bekannt ist, bildet sich bei der Implantation von PAAG im Organismus des Säugetiers um das Gel herum eine Verbindungskapsel (A.B. Shekhter et al., "Injectable hydrophilic polyacrylamide gel Formaciyl and tissue response to its implantation", in der Fachzeitschrift "Annalen der plastischen, rekonstruktiven und ästhetischen Chirurgie", 1997, Nr. 2, Seite 19), die für eine gewisse Zeit das Eindringen von T-Lymphozyten zu den im PAAG implantierten Zellen und die Zerstörung der implantierten Zellen verhindert.

Jedoch nicht nur das Vorhandensein der gewebeverbindenden Kapsel beeinflusst die Dauer der Erzeugung der durch die heterogenen Zellen veranlassten, für den Organismus des Kranken erforderlichen Stoffe.

Durch die EP 742022 ist ein bioverträgliches Polyacrylamid-Hydrogel bekannt, das von 3,5 bis 9,0 Mass.% des Kopolymers Acrylamid mit dem Vernetzungsagens Methylen-bis-Acrylamid und 96,5-99,0 Mass.% Wasser enthält.

Dieses Hydrogel wird durch ein Verfahren gewonnen, das im gleichen Dokument beschrieben ist (EP 742022) und das darin besteht, das die Reaktion der Kopolymerisation von Acrylamid mit Methylen-bis-Acrylamid im Wasser in Anwesenheit von Peroxidinitiatoren der Polymerisation mit Verzögerung der Reaktionsmischung bei Zimmertemperatur innerhalb von 20 Minuten für die Vernetzung des Polymers durchgeführt wird. Dabei wird der Prozess der Kopolymerisation in einer Stufe durchgeführt. Als Peroxid-Polymerisationsinitiatoren wird ein Gemisch aus Peroxodisulfat-Ammonium und Tetramethyläthylendiamin verwendet. Als Wassermedium wird Apyrogenwasser oder eine Natriumchloridlösung genommen.

Das Hydrogel, das mit diesem Verfahren gewonnen wurde, hat einen nicht ausreichenden Vernetzungsgrad, was durch das niedrige Temperaturverhalten bei der Durchführung des Kopolymerisationsprozesses und die Einstufigkeit des Verfahrens bedingt ist. Dies führt zu einer schnellen Durchwachsung des Bindegewebes in das implantierte Gel und zu dessen schnellem Schrumpfen und schneller Resorption (A.B.Shekhter et all "Injectable hydrophilic polyacrylamide gel Formacryl and tissue response to its implantation", in der Fachzeitschrift "Annalen der plastischen, rekonstruktiven und ästhetischen Chirurgie", 1997, Nr. 2, Seite 19).

Außerdem enthält das mit diesem Verfahren gewonnene Hydrogel nichtverbundene Moleküle von Tetramethyläthylendiamin, freie NH₂-Radikale und Monomere von Acrylamid in einer Menge von 1,0-1,2mkg auf 1 Gramm Polymer (1,0-1,2ppm), wodurch eine aktive, aseptische und entzündliche Reaktion im Frühstadium der Einführung des Hydrogels in den Organismus hervorgerufen werden kann (s. A. B. Shekhter et al "Injectable hydrophilic polyacrylamide gel Formacryl and tissue response to its implantation", in der Fachzeitschrift "Annalen der plastischen, rekonstruktiven und ästhetischen Chirurgie ", 1997, Nr. 2, Seite 19).

Durch das Patent RU 2127129 ist ein bioverträgliche Hydrogel bekannt, das von 1,0 bis 8,0 Mass.% quervernetztes Kopolymer von Acrylamid mit dem Vernetzungsagens Methylen-bis-Acrylamid und 92,0-99,0 Mass% Wasser enthält. Ein Verfahren zur Gewinnung dieses Materials ist auch im Patent RU 2127129 beschrieben und besteht in der Kopolymerisation von Acrylamid mit Methylen-bis-Acrylamid im wässrigen Dispersionsmittel in Anwesenheit eines Peroxid-Polymerisationsinitiators. Dabei wird als Wassermedium der Elektrolyse untergezogenes Wasser mit einem pH-Wert von 9,0-9,5 genommen. Die Vernetzung des Kopolymers wird bei der Inkubation der Reaktionsmischung in zwei Stufen durchgeführt; und zwar bei einer Temperatur von 20-90°C innerhalb von 2-24 Stunden und dann bei einer Temperatur von 100-105°C im Laufe von 2-4 Stunden.

Das mit diesem Verfahren gewonnene Hydrogel enthält kein Tetramethyläthylendiamin, enthält jedoch 1% freie NH₂-Radikale und Monomere des Acrylamids in einer Menge von 0,6-0,8mkg auf 1 Gramm des Polymers (0,6-0,8ppm). Doch dieses Hydrogel ist vorwiegend für die Plastik von weichen Geweben entwickelt und geeignet und sichert keine ausreichende Dauer der aktiven Funktion der heterogenen Zellen bei seiner Verwendung als Träger der implantierten Zellen.

Durch die US 5 338 492 ist ein Polyakrylamid-Hydrogel für Bestrahlungs-farbfilter bekannt. Das Hydrogel weist ein quervernetztes Kopolymer des Akrylamids (50-70 Gew.-%) mit Hydroxyethylmethakrylsäureester und Quervernetzungsmitteln, nämlich Methylen-bis-akrylamid oder Methylen-bis-methakrylamid, auf, wobei ein Gewichtsverhältnis des Quervernetzungsmittels zum Akrylamid von etwa 1:5 bis etwa 1:12 verwendet wird. Bevorzugt werden Gewichtsverhältnisse von etwa 1:8 bis etwa 1:12. Besonders bevorzugt wird ein Gewichtsverhältnis von etwa 1:9. Der Feststoffanteil liegt vorzugsweise im Bereich von etwa 9 Gew.-%. Dieses Hydrogel wird unter Verwendung einer einstufigen Reaktion präpariert, wobei Wasserlösungen der entsprechenden Monomere mit den Quervernetzungsmitteln gemischt werden, die Polymerisierungsstarter Benzoinmethylether, Benzoinethylether, Bernstein-säure-Peroxid, 2-Hydroxy-2-methyl-1-phenylpropan-1-one, 4-(2-Hdroxyethoxy)-phenyl-(2-propyl)-Keton, 2,2'Azo-bis-(2,4-dimethyl-4-methoxy-valero)-nitril oder Azo-bis-isobutyonitril verwendet werden und organische Lösungsmittel (Isopropylalkohol oder Ethylenglykol) zugefügt werden, die darauf abzielen, die Dispersion des Starters in der wässrigen Lösung zu verbessern. Dieses Hydrogel ist für Zellen und Gewebe giftig, und seine Verwendbarkeit ist auf den technischen Gebrauch beschränkt. Wegen der einstufigen Polymerisierung weist es ferner nur einen geringen Grad an Quervernetzung auf.

Durch die WO 0 216 453 ist ein Polyakrylamid-Hydrogel bekannt, das als injizierbares oder implantierbares, endoprosthetisches Hilfsmittel eingesetzt wird und quervernetztes Polyakrylamid aufweist. Dieses Hydrogel wird dadurch erzeugt, dass Akrylamid und N,N'-methylen-bis-akrylamid kombiniert und zur Quervemetzung in einem besonderen Gewichtsverhältnis verwendet werden. Diese Hydrogel wird dadurch erzeugt, dass eine wässrige Monomerlösung des Akrylamids (AM) und des N,N'-methylen-bis-Akrylamids (BISAM) als Quervernetzer mit N,N,N',N'-tetramethylenethylendiamin (TMED) als Kostarter und Ammoniumpersulfat (APS) als radikalloser Starter (Redox-System) gemischt werden. Durch ein Entgasen einer umfangreichen Lösung mit Stickstoff (auf einer Temperatur von 45 °C gehalten und während einer Dauer von 20 s mit Stickstoff entgast) startet die Polymerisierung (die für eine Dauer von 0,5-1,5 Stunden erfolgt). Nach einer abschließenden Polymerisierung wird das Gel in einen Waschtank mit Netzschalen gefüllt, in die das Gel aufgenommen wird. Während des Waschens des Gels mit Wasser dehnt sich das Gel aus, und Monomerreste werden unter Gleichgewichtsherstellung mit Wasser für eine Dauer von 92 Stunden abgezogen, wobei das Wasser mehrere Male ausgetauscht wird. Das erzeugte Hydrogel weist dieselben Nachteile wie die des Hydrogels auf, das in der EP-Anmeldung 742022 beschrieben ist. Das Hydrogel weist einen ungenügenden Grad an Quervernetzung auf, der auf die Tieftemperaturbedingungen des Kopolymerisierungsprozesses und dessen einstufige Durchführung zurückzuführen ist, so dass ein schnelles Eindringen des Bindegewebes in ein implantiertes Hydrogel und ein schnelles Schrumpfen und eine schnelle Resorption des Hydrogels erfolgen können. Dieses Hydrogel kann ungebundene Moleküle des Tetramethylethylendiamins, freie NH₂-Radikale und Akrylamid-Monomere aufweisen, so dass eine aktive, aseptische Entzündungsreaktion unmittelbar nach der Implantation des Hydrogels im Organismus erfolgen kann (s. A. B. Shekhter et al., "Interjectable hadrophilic polyacrylamide gel Formacryl and tissue response to its implantation" in "Annals of plastic, reconstructive and esthetic surgery", 1997, Nr. 2, Seite 19).

Ferner sind durch den Artikel von Olga García et al. "5-Fluorouracil trapping in poly(2-Hydroxyethyl methacrylate-coacrylamide) hydrogels: in vitro drug delivery studies", European Polymer Journal, Vol. 36, 2000, Nr. 1, Seiten 111-122, Polyakrylamid-Hydrogele bekannt, die Kopolymer-Akrylamid, 2-Hydroxyethylmethakrylat (HEMA) und Ethylenglykoldimethakrylat (EGDMA) aufweisen. Das Verfahren zur Erzeugung dieses Gels umfasst die Gewinnung einer Wassermischung der oben beschriebenen Komponenten, das Zufügen von Ammoniumperoxodisulfat und die Entgasung der Mischung mittels Stickstoffs für die Dauer von 5 Minuten. Dann wird die Mischung drei Stunden lang auf einer Temperatur von 50 °C (323 °F) gehalten. Die Verwendung von Ethylenglykoldimethakrylat (EGDMA) als Quervernetzungsmittel) erlaubt die Gewinnung eines sich stark ausdehnenden Hydrogels, das durch Makrophagen leicht resorbiert werden kann, wenn das Hydrogel in den Empfängerorganismus implantiert ist. Ferner weist dieses Hydrogel einen ungenügenden Grad an Quervemetzung auf, der auf die einstufige Durchführung des Polymerisierungsprozesses zurückzuführen ist und die ein schnelles Eindringen des Bindegewebes in das implantierte Gel und eine schnelle Schrumpfung und Resorption des Hydrogels zur Folge haben kann.

Aus der WO 0 045 792 ist schließlich ein ätzendes Polyakrylamid-Hydrogel bekannt, das quervernetzt ist und durch Kopolymerisieren von Akrylamid mit N,N'-Methylen-bis-akrylamid gebildet ist. Die gewöhnlichen Hydrogelmonomere weisen 1-Hydroxyethylmethakrylat (HEMA) auf. Die gewöhnlichen Quervernetzungsmittel umfassen Tetraethylenglykoldimethakrylat (TEGDMA) und N,N'-Methylen-bis-akrylamid. Dieses Hydrogel kann ebenfalls nicht eine lange Lebensfähigkeit der heterogenen Zellen im Empfängerorganismus aufgrund einer schnellen Zersetzung im lebenden Organismus garantieren, wie ausdrücklich in diesem Dokument dadurch bestätigt wird, dass erosionsanfällige Hydrogele, bei denen eine hydrolytische Instabilität in den Quervernetzungen auftritt, ein quervernetztes Produkt aufweisen, das durch Kopolymerisieren von N-Vinylpyrrolidon oder Akrylamid mit N,N'-Methylen-bis-Akrylamid hergestellt wird. Die Verwendung von Tetraethylenglykoldimethakrylat (TEGDMA) als Quervernetzungsmittel ergibt eine steigende Wahrscheinlichkeit einer ausgesprochenen Gewebereaktion, die durch die unvermeidbare Gegenwart von Restanteilen der TEGDMA-Monomere verursacht wird.

Die Hauptaufgabe, die mit der vorgeschlagenen Erfindung gelöst werden soll, ist die Steigerung der aktiven Lebensfähigkeit der im Polyacrylamid untergebrachten, heterogenen Zellen im Organismus des Empfängers.

Eine weitere Aufgabe besteht in der Senkung des Resorptionsgrads des Hydrogels und der Möglichkeit des Eindringens von Makrophagen nach Implantierung des Hydrogels in den Organismus des Empfängers.

Eine weitere Aufgabe ist die Verminderung der Gewebereaktion des Organismus auf das implantierte Hydrogel durch die Verminderung von freien Radikalen und Monomeren.

Die gestellten Aufgaben werden dadurch gelöst, dass ein mehrfunktionelles, bioverträgliches Hydrogel vorgeschlagen wird, das Polyacrylamid und Wasser enthält, wobei gemäß der Erfindung das Polyacrylamid das Kopolymer von Acrylamid, Methacrylamid, 2-Hydroxiäthyl-Metacrylat und N,N'-Methylen-bis-Acrylamid aufweist.

Das genannte Polyacrylamid enthält die folgenden Verhältnisse der Komponenten in Mass.%:

| | |
|---|---|
| Acrylamid | 65,0-80,0, |
| Methacrylamid | 18,0-30,0, |
| 2-Hydroxyäthil Metacrylat | 0,1-4,0 |
| N,N'-Methylen-bis-Acrylamid | 0,2-6,0. |

Das genannte Polyacrylamid beträgt 3,0 bis 10,0 Mass.% der gesamten Masse des bioverträglichen Hydrogels.

Das genannte, bioverträgliche Hydrogel enthält folgende Verhältnisse der Komponenten in Mass.%:

| | |
|---|---|
| Acrylamid | 1,95-8,0, |
| Methacrylamid | 0,54-3,0, |
| 2-Hydroxyäthyl Metacrylat | 0,003-0,4, |
| N,N'- Methylen-bis-Acrylamid | 0,006-0,6, |
| Wasser | bis 100. |

Als Wasser enthält das Hydrogel bidestilliertes Apyrogenwasser. Das mehrfunktionelle, bioverträgliche Hydrogel weist einen pH-Wert von 3,5-4,5 auf.

Das genannte Hydrogel ist für die Injektion geeignet, in Spritzen abgepackt und für die Bildung der Kapsel im Organismus von Tieren und Menschen gut geeignet. Dazu wird es in den Organismus implantiert, und dann wird darin die gewünschte Zellkultur untergebracht.

Die gestellten Aufgaben werden folgendermaßen gelöst. Es wird ein Verfahren zur Gewinnung eines mehrfunktionellen, bioverträglichen Hydrogels durch Kopolymerisation von Monomeren und eines Vernetzungsagens in wässrigem Medium in Anwesenheit von Peroxidinitiatoren vorgeschlagen. Dieses Verfahren läuft in mehreren Stufen ab. Bei diesem Verfahren werden gemäss der Erfindung als Monomere Acrylamid und Methacrylamid genommen, und als Vernetzungsagens wird Hydroxiäthyl-Metacrylat und N,N'-Methylen-bis-Acrylamid bei folgenden Verhältnissen der Komponenten in Mass.% genommen:

| | |
|---|---|
| Acrylamid | 1,95-8,0, |
| Methacrylamid | 0,54-3,0, |
| 2- Hydroxiäthyl Metacrylat | 0,003-0,4, |
| N,N'- Methylen-bis-Acrylamid | 0,006-0,6, |
| Wasser | bis 100. |

Die Kopolymerisation wird in drei Stufen durchgeführt: die erste Stufe erfolgt bei Zimmertemperatur von 20-30°C innerhalb von 12-24 Stunden, die zweite Stufe erfolgt durch γ-Bestrahlung mit einer Dosis von 0,4-1,0 Megarad, und die dritte Stufe erfolgt bei einer Temperatur von 100-130°C und einem Druck von 0-1,2 Atmosphären innerhalb von 20-40 Minuten.

Das genannte Hydrogel wird nach der ersten Stufe der Kopolymerisation mit heißem Wasser, das eine Temperatur von 70-110°C hat, mindestens 3 Stunden lang bei einem Massenverhältnis des Hydrogels zum Wasser von 1:8-10 und einem Druck von 0-1,2 Atmosphären abgespült.

Als Kopolymerisationsinitiator wird Wasserstoffperoxyd und/oder Peroxydsulfat-Ammonium in einer Menge von nicht mehr als 0,33% Mass.% des summarischen Gewichts der Ausgangskomponenten genommen. Als wässriges Medium wird bidestilliertes Apyrogenwasser genommen.

Das gewonnene Hydrogel wird in Spritzen gefüllt bzw. abgepackt und für die Implantierung in den Organismus von Tieren und Menschen unter Bildung einer Kapsel verwendet. Danach werden die gewünschten Zellkulturen in die Kapsel eingeführt.

Das vorgeschlagene Hydrogel kann mit unterschiedlichen Verfahren gewonnen werden, und das Verfahren gemäß der Erfindung zur Gewinnung von Polyacrylamid-Hydrogel beschränkt die anderen möglichen Varianten der Gewinnung, sowohl der direkten als auch indirekten, des Hydrogels nicht. Dieses Verfahren stellt einen der möglichen Wege zur Gewinnung des Hydrogels dieser Zusammensetzung mit vorher festgelegten Eigenschaften dar.

Wie bekannt ist, stellt das Hydrogel, das Polyacrylamid enthält, ein dreiachsiges Netz der mit dem Vernetzungsagens quervernetzten Monomere dar, in dessen Zellen das wässrige Medium enthalten ist. In diesem wässrigen Medium ist eine nicht festgelegte Menge von nichtverbundenen Polymerisationsinitiatoren enthalten, da eine gewisse, auch nicht festgelegte Menge von Polymerisationsinitiatoren unmittelbar in die Struktur des Kopolymers eingebaut (s. M.N. Sawitzkaya, Ju.D.Holodova "Polyacrylamid", Verlag "Technik" 1969, S. 103) oder aus dem Hydrogel bei dessen Abspülen ausgewaschen wird.

Dabei hängen die biologisch aktiven Eigenschaften eines solchen Gels wesentlich von der Struktur des Netzpolymers ab, die ihrerseits von den Bedingungen für die Synthese des Netzpolymers abhängt, und zwar vom qualitativen und quantitativen Verhältnis der Ausgangsreagenzien, darunter das Vernetzungsagens und die Polymerisationsinitiatoren, die mit ihren chemischen und WasserstoffVerbindungen in die Struktur des Polymers eingebaut werden (nach Gruppen NH, CH, COOH, NH_{2,} CH₂). Diese Eigenschaften hängen auch von der Art der Polymerisation ab.

Das Wesen der Erfindung besteht darin, dass eine qualitative und quantitative Zusammensetzung des Polyacrylamid-Gels sowie die Bedingungen der Durchführung der Kopolymerisation gefunden wurde, die die Dauer der aktiven Lebensfunktion der in den Organismus implantierten, heterogenen Zellen zu erhöhen erlauben.

Die Bedingungen zur Gewinnung des vorgeschlagenen Polyacrylamid-Hydrogels haben erlaubt, die Anzahl der nicht verbundenen Aminogruppen, der freien NH2-Radikale sowie der ungesättigten Doppelbindungen zu vermindern. Es ist auch gelungen, den Vernetzungsgrad durch die Bildung von Strukturgruppen (H₂C-NH-CH₂-), (-CO-NH-CR₂-O-R), (-CO-NH-NH-CO-), (H-COR-NH-CR-O-R), (-CONH-R-NH-CO), wobei R gleich CH₃, CH₂, NH₂, C₂H₅ oder C₃H₇ ist, sowie durch die Erhöhung der Anzahl der Quervernetzungen der N-N-Bindungen zu erhöhen.

Dadurch können eine hohe Beständigkeit des vorgeschlagenen Hydrogels in Bezug auf die Resorption und das Schrumpfen im Organismus des Patienten sowie Bedingungen für das Überleben der ins Gel implantierten Zellen gewährleistet werden.

### Kurzfassung der Zeichnungen

Die Erfindung wird nun anhand von Ausführungsbeispielen näher erläutert. Es zeigen:
- Fig. 1a: ein infrarotes (IR) Absorptionsspektrum des vorgeschlagenen Gels,
- Fig. 1b: ein infrarotes (IR) Absorptionsspektrum eines Prototyps des Gels, der in Russland nach dem Patent RU 2127129 unter der Handelsmarke "Formacryl" hergestellt wird, wobei beide IR-Spektren im Bereich von 4000-500cm⁻¹ liegen, auf der X-Achse die Länge der Lichtwelle (cm⁻¹) und auf der Y-Achse der Grad der Lichtabsorption T (in %) angegeben ist,
- Fig. 2a: ein Photo des vorgeschlagenen Hydrogels, das mit einem elektronischen Rastermikroskop aufgenommen wurde, und
- Fig. 2b: ein Bild des Hydrogels "Formacryl", das ebenfalls mit einem elektronischen Rastermikroskop aufgenommen wurde.

Zur Gewinnung des vorgeschlagenen, bioverträglichen Gels wird folgendes genommen:
- Acrylamid (acrylamide): C₃H₅NO₃, Mol-Masse 71.08, weißes Kristallpulver ohne Geruch, Schmelztemperatur 84,5°C, Produktion der Firma Sigma (Katalog "Reagenzien für die Biochemie und Untersuchungen im Bereich der Naturwissenschaften", SIGMA, 1999, S. 47, Katalognummer Nr. A8887),
- Methacrylamid (methacrylamide): C₃H₇NO, Mol-Masse 73.08, weißes Pulver, Schmelztemperatur 111°C, Produktion der Firma Fluka (Fluka Katalogue "Chemica-Biochemica", Fluka AG, Switzerland, 1986/87, P-1151),
- 2-Hydroxyethyl-Methacrylat (2hydroxyethylmethacrylate): C₆H₁₀O₃, Mol-Masse 130.1, Flüssigkeit, Siedetemperatur 205-208°C, Dichte 1,07g/ml, Produktion der Firma Sigma (Katalog "Reagenzien für die Biochemie und Untersuchungen im Bereich der Naturwissenschaften", SIGMA, 1999, S. 567, Katalognummer Nr. H8633),
- N,N'-Methylen-bis-Acrylamid (N,N'-methylene-bis-acrylamide): C₇H₁₀N₂O₂, Mol-Masse 154,16, weißes Kristallpulver ohne Geruch, Schmelztemperatur 185°C, Produktion der Firma Sigma (Katalog "Reagenzien für die Biochemie und Untersuchungen im Bereich der Naturwissenschaften", SIGMA, 1999, S. 696, Katalognummer Nr. 7256),
- Peroxodisulfat-Ammonium: (NH₄)₂-S₂O₈, Mol-Masse 228.19, farblose, flache Kristalle, Zerstörungstemperatur 120°C, Produktion der Firma Sigma (Katalog "Reagenzien für die Biochemie und Untersuchungen im Bereich der Naturwissenschaften", SIGMA, 1999, S. 117),
- Wasserstoffperoxid : H₂O₂, Mol-Masse 34,0, farblose Flüssigkeit, Dichte bei 0°C: 1,465, Schmelztemperatur 0,89°C, Produktion der Firma Sigma (Katalog "Reagenzien für die Biochemie und Untersuchungen im Bereich der Naturwissenschaften", SIGMA, 1999, S. 556, Katalognummer H6520).

Alle oben genannten Monomere gelten als geeignet für die biologischen Ziele, sie brauchen keine biologische Reinigung. Als Wasser wird bidestilliertes Apyrogenwasser (pH-Wert 5,6) genommen.

Das Verfahren wird folgendermaßen realisiert:

Für die Vorbereitung der Reaktionsmischung wird bidestilliertes Apyrogenwasser mit einem pH-Wert von 5,6 genommen. Eine Wasserlösung wird zubereitet, die Acrylamid enthält, wobei Methacrylamid, 2-Hydroxyethyl-Methacrylat und N,N'-Methylen-bis-Acrylamid im Verhältnis 65,0-80,0:18,0-30,0:0,1-4,0:0,2-6,0 zueinander stehen. Die Gesamtmasse der Ausgangsmonomere in der Lösung beträgt dabei 3,0 bis 10,0% (durch das Variieren der Ausgangsmonomere in der Lösung wird ein Hydrogel unterschiedlicher Dichte und Elastizität gewonnen). In die gewonnene Lösung werden die Polymerisationsinitiatoren Wasserstoffperoxid in der Menge von 0,1-0,3 Mass.% oder Peroxodisulfat-Ammonium in der Menge von 0,0006-0,03 Mass,% oder ein Gemisch daraus im beliebigen Verhältnis und in einer Menge eingeführt, die die Summe ihrer maximalen Werte nicht überschreitet. Durch das Variieren der Menge von Wasserstoffperoxid und Peroxodisulfat-Ammonium wird Material gewonnen, das den gewünschten pH-Wert im Bereich von 3,5-4,5 hat. Die fertige Reaktionsmischung wird durch bakterizide Polymerfilter gefiltert, beispielsweise vom Typ F8273 mit einer Porengröße von 0,45mm, CA/CN, Produktion Sigma (USA). Die Mischung wird für die Kopolymerisation der Monomere zur Inkubation bei einer Temperatur von 20-30°C innerhalb von 12-24 Stunden gebracht. Nach dieser Inkubation wird das Hydrogel, das schon als Gel aussieht, mit heißem Wasser abgespült. Dazu wird das Gel in einen Behälter mit Wasser, das eine Temperatur von 70-110°C aufweist, bei einem Druck von 0-1,2 Atmosphären und einem Verhältnis des Gel-Anteils zu Wasser von 1:8-10 für 4-6 Stunden gebracht. Dann wird die zweite Stufe der Kopolymerisation ausgeführt, wobei das Halbprodukt mit einer γ-Bestrahlung in einer Dosis von 0,4-1,0 Megarad bearbeitet wird. Danach wird das Produkt im erforderlichen Umfang in Flakons oder in Spritzen abgefüllt. Danach wird die dritte Stufe der Kopolymerisation durchgeführt, wobei das Gel einer Temperatur von 120°C und einem Druck von 1,2 Atmosphären innerhalb von 20-40 Minuten ausgesetzt wird.

Es wurden physisch-chemische, hygienisch-chemische und toxikologische Untersuchungen von Mustern des vorgeschlagenen Hydrogels in Übereinstimmung mit dem Standard ISO 10993 "Auswertung der biologischen Wirkung von medizinischen Erzeugnissen", "Methodische Hinweise zu der hygienischen Auswertung der Gummi- und Latexerzeugnisse für medizinische Zwecke" (Gesundheitsministerium UdSSR, M., 1988) und in Übereinstimmung mit den methodischen Empfehlungen "Zulässige Mengen der Migration von chemischen Stoffen, die sich aus Polymer- und anderen Werkstoffen aussondern und die mit Lebensmittelhydrogelen kontaktieren, sowie die Verfahren für ihre Bestimmung", SanPiN (CaH 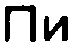 H) 42-122-42-40-86, durchgeführt.

Die Bestimmung des Gehalts an den Monomeren Acrylamid und N,N'-Methylen-bis-Acrylamid in den Mustern des gewonnenen Hydrogels wurde in Übereinstimmung mit der Methodik durchgeführt, die in der Arbeit von V.V. Kuznetsov et al., "Determination of Acrylamide in Polyacrylamidic gels", The 52-nd is Pittsburgh Conference on Analytical Chemistry and Applied Spectroscopy, New Orleans, LA, 2001, Abstract Book, Nr. 1648, beschrieben ist.

Bei dieser Untersuchung wurde festgestellt, dass das vorgeschlagene Hydrogel folgende physisch-chemische Eigenschaften besitzt:
- Aussehen: Gel
- Farbe: von farblosem bis zum halbdurchsichtigen Dunkelbraun, opalisierend,
- Brechungsverhältnis: 1,328-1,360,
- Dichte: 1,0-1,2g/cm³,
- pH-Wert: 3,5-4,5,
- Gehalt an Monomeren: bis 0,4ppm,
- Bromierungsniveau: nicht mehr als 1,0 (mg Brom je 1I).

Hygienische Versuche haben gezeigt, dass die Migration der Metalle Cu, Fe, Ni, Zn, Al, Ti, Ag in einem wässrigen Extrakt aus Hydrogel, die mit dem Atomabsorptionsverfahren bestimmt wird, in den Grenzen der Empfindlichkeit des Verfahrens nicht gefunden wurde (jeweils 0.02, 0.05, 0.05, 0.02, 0.005 und 0.04mg/l). Diese Werte sind wesentlich niedriger als die zugelassenen Werte, die für das Trinkwasser vorgeschrieben sind. Die Migration von Natrium betrug nicht mehr als 0,12 mg/l bei einem zulässigen Niveau im Trinkwasser von 200 mg/l.

Die technologischen Versuche haben gezeigt, dass die wässrigen Extrakte aus Hydrogel keinen hämolytischen Effekt in den Versuchen "in vitro" mit den mittels Erythrozyten isolierten Kaninchen aufgewiesen haben. Es wurde eine Hämolyse von 0,04% bei einem zulässigen Wert von 2% festgestellt.

Bei einem scharfen Versuch mit weißen Mäusen bei der parenteralen Einführung der Hydrogel-Muster in einer Dosis von 50,0ml auf 1 kg Körpermasse wurde der Tod der Tiere sowie die klinischen Merkmale der Intoxikation nicht festgestellt: der allgemeine Zustand der Versuchsmäuse, ihr Verhalten, die Futtereinnahme und der Zustand des Fells unterschieden sich von den Kontrollzuständen nicht. Bei der Obduktion der Versuchsmäuse wurde festgestellt, dass das Gewebe an der Stelle der Einführung des Hydrogels die regionalen Lymphknoten und Innenorgane (Leber, Nieren, Milz) in den Grenzen der physiologischen Norm und Kontrolle waren. Es wurden keine statistisch zuverlässigen Unterschiede bei der Dynamik der Körpermasse, der klinisch-biochemichen Blutwerte, der Kennziffer der Innenorgane bei den Versuchstieren im Vergleich zu den Kontrollwerten bei der Unterhautimplantierung des Gels für 2,5 Monate festgestellt.

Es wurde kein Sensibilisierungseffekt des Hydrogels bei der Durchführung der immunologischen Diagnosereaktion bei der Degranulierung der Mastzellen ermittelt. Bei einem Mikrokernversuch auf dem Präparat des Knochenmarks wurde kein mutagener Effekt der Hydrogel-Wirkung festgestellt. Die feingewebliche Untersuchung des Bereichs der Hydrogel-Implantierung und der Innenorgane (Leber, Nieren, Milz, Samenblase) zeigte das Vorhandensein einer schwach ausgeprägten Gewebereaktion auf Hydrogel nur in den ersten Tagen nach der Implantierung sowie das Fehlen von dystrophischen und nekrotischen Veränderungen in den Organen.

Im Folgenden sind konkrete Beispiele zur Gewinnung des vorgeschlagenen, bioverträglichen Hydrogels und seiner Anwendung für die Kultivierung von heterogenen Zellen im Organismus des Empfängers angegeben.

### Beispiel 1

Zur Gewinnung von Hydrogel wurden 384ml bidestilliertes Apyrogenwasser mit einem pH-Wert von 5,6 genommen. Im Wasser wurden dann 11,2g Acrylamid, 3,6g MethAcrylamid, 0,48g 2-Hydroxyethyl-Methacrylat und 0,72g N,N'-Methylen-bis-Acrylamid genommen, die für biologische Zwecke geeignet sind. Danach wurden in die Ausgangslösung 0,04g Peroxodisulfat-Ammonium und 2ml 30% iges Wasserstoffperoxid hinzugefügt. Die gewonnene Mischung wurde durch ein bakterizides Polymerfilter gefiltert, Typ F8273 mit einer Porengröße von 0,45mm, CA/CN, Hersteller Fa. Sigma (USA). Die Mischung wurde in einen Behälter gefüllt, der zur Inkubation im Wasserbad bei einer Temperatur von 30°C innerhalb von 22 Stunden gebracht wurde.

Danach wurde das Hydrogel in Form des Gels mit heißem Wasser im Verhältnis Wasser zu Gel von 10:1 bei einer Temperatur von 90°C innerhalb von 4 Stunden abgespült. Dann wurde die zweite Stufe der Kopolymerisation durchgeführt, wobei das Halbprodukt mit einer γ-Bestrahlung in einer Dosis von 0,8 Megarad bearbeitet wurde. Danach wurde das Produkt in Spritzen von 0,5-3,0ml abgefüllt. Dann wurde die dritte Stufe der Kopolymerisation durchgeführt, wobei das Gel einer Temperatur von 120°C und einem Druck von 1,2 Atmosphären 30 Minuten lang ausgesetzt wurde.

Das gewonnene Gel enthält 96 Mass.% der Wassephase und 4 Mass.% Kopolymer, wobei auf 70,0 Mass.% Acrylamid 22,5 Mass.% Methacrylamid, 0,3 Mass.% 2-Hydroxyethyl-Methacrylat, 0,4 Mass.% N,N'-Methylen-bis-Acrylamid entfallen. Das Gel weist einen pH-Wert von 4,3 auf.

Das gewonnene Muster des Hydrogels hat folgende physisch-chemische Eigenschaften:
- Aussehen: farbloses, halbdurchsichtiges, opalisierendes Gel,
- Brechungsverhältnis: 1,348,
- PH-Wert: 4,3,
- Dichte: 1,0g/cm³,
- Gehalt an Monomeren: 0,1 ppm,
- Bromierungsniveau: 0,1 (mg Brom auf 1I).

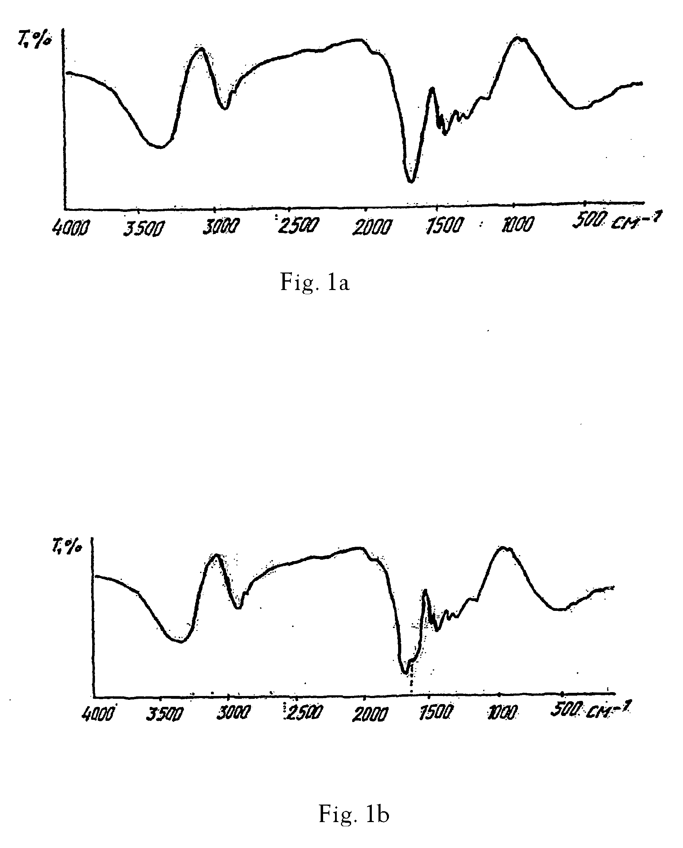

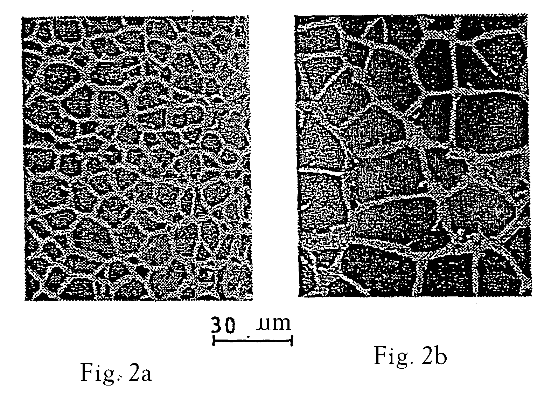

Es wurde das IR-Spektrum erhalten, und es wurden elektronenmikroskopische Untersuchungen des ausgetrockneten Musters dieses Hydrogels durchgeführt, die jeweils in den Figuren 1a und 2a dargestellt sind. Zum Vergleich sind in den Figuren 1b und 2b das infrarote Spektrum und das Chromatogramm des Extrakts des berühmten Hydrogel-Prototyps, der in Russland nach dem Patent RU 2127129 unter der Handelsmarke "Formacryl" hergestellt wird, dargestellt. Dieses Gel enthält 96 Mass.% der Wasserphase und 4 Mass.% Kopolymer, wobei auf 96 Mass.% der Wasserphase 4,0 Mass% N,N'-Methylen-bis-Acrylamid mit einem pH-Wert von 5,4 und einem Bromierungsniveau von 0,27 (mg Brom auf 1l) entfielen. Das Gel wurde bei der Inkubation der Ausgangsmischung in Anwesenheit von Wasserstoffperoxid und Peroxodisulfat-Ammonium in der summarischen Menge von 0,3 Mass% bei einer Temperatur von 60°C innerhalb von 12 Stunden und dann bei einer Temperatur von 100°C noch innerhalb von 2 Stunden erhalten.

Wie aus dem Spektrum in Fig. 1 a zu ersehen ist, fehlt die Spitze im Bereich des Streifens bei 1620cm⁻¹. Dies zeugt davon, dass in der Struktur des Polymers die freien NH₂-Radikale fehlen, die die Koordinierungsverbindungen mit dem Strukturnetz des Hydrogels bilden konnten.

Wie aus dem Spektrum in Fig. 1b zu ersehen ist, gibt es einen Streifen bei 1620 cm⁻¹. Dies weist auf das Vorhandensein von NH₂-Radikalen in einer Menge von etwas mehr als 1% hin.

Zur Durchführung der elektronenmikroskopischen Untersuchungen des angemeldeten Hydrogels und des Hydrogels "Farmokril" wurden die Muster des Gels, die nach dem beschriebenen Beispiel gewonnen wurden, bis zu einem konstanten Gewicht ausgetrocknet. Dabei haben die Muster die Form eines Films erhalten. Die Struktur der Hydrogele wurde durch das Verfahren der elektronischen Rastermikroskopie (REM) unter Verwendung eines Elektronenmikroskops S 405A untersucht. Dazu wurde die Präparation durch das Verfahren der Absplitterung der in flüssigem Stickstoff eingefrorenen, ausgetrockneten Muster der Hydrogele durchgeführt. Bevor das Verfahren der elektronischen Rastermikroskopie zum Einsatz kam, wurde Gold auf die Oberfläche der Muster aufgestäubt.

Die elektronenmikroskopischen Bilder des vorgeschlagenen Hydrogels und des Hydrogels "Formakril" sind jeweils in den Figuren 2a und 2b dargestellt. Wie sich aus dem Vergleich der in Fig. 2 dargestellten Bilder erkennen lässt, hat das vorgeschlagene Hydrogel eine Feinzellenstruktur im Vergleich zum Hydrogel "Formakril".

### Beispiel 2

Das vorgeschlagene Hydrogel wird zur Kultivierung von xenogenen Geschwulstzellen verwendet.

Das nach dem beschriebenen Beispiel 1 gewonnene Hydrogel wurde subkutan in einer Menge von 1 ml Mäusen der Linie C57-Black implantiert. Nach dem Verlauf von 1,5 Monaten wurden in das implantierte Gel, um das sich eine Bindegewebskapsel gebildet hatte, menschliche Melanomzellen der Linie SKMEL-1, und zwar je 1 Mio. Zellen auf jede Kapsel mit Hydrogel eingebracht. Nach 3, 6 und 9 Monaten wurden die Kapseln aus den Tieren herausgenommen und die Existenzfähigkeit und die physiologische Aktivität der in diesen Kapseln befindlichen Melanomzellen des Menschen geprüft.

Es wurde festgestellt, dass die Melanomzelle des Menschen der Linie SKMEL-1 ihre Existenzfähigkeit sogar 9 Monate nach der Kultivierung in der Kapsel des vorgeschlagenen Hydrogels aufrecht erhalten hatte. Bei der Überführung der aus der Kapsel ausgesonderten Melanomzellen in die Kultur wurde mit dem konventionellen Verfahren der Polymerase-Kettenreaktion die volle Identität des konservierten Mioglobins des Menschen in den Melanomzellen, die im Nährboden kultiviert sind, sowie die Identität der Zellen festgestellt, die innerhalb von 9 Monaten in der Kapsel des Hydrogels kultiviert wurden, die in den Organismus einer Maus implantiert waren.

Dieses Beispiel zeugt von der Möglichkeit, mit dem vorgeschlagenen Hydrogel die in den Organismus des Empfängers implantierten Zellen auf Dauer zu kultivieren, wobei die Zellen ihre Existenzfähigkeit aufrecht erhalten.

### Beispiel 3

Das vorgeschlagene Hydrogel wird zur Kultivierung von Leiding-Schweinezellen im Organismus des Menschen zur Behandlung der Unfruchtbarkeit verwendet.

Das Hydrogel, das nach dem beschriebenen Beispiel 1 gewonnen wurde, wurde kranken Menschen männlichen Geschlechts implantiert. Nach der Bildung der Bindegewebskapsel um das Gel wurden in das Gel die Zellen eingebracht, die von geschlechtsreifen Ferkeln genommen wurden.

Das Testosteronniveau im Blut des Patienten wurde mit Hilfe eines diagnostischen Satzes der Fa. ChemaMedika (Russland) nach der Anweisung des Herstellers. ermittelt. Das Testosteronniveau beim Patienten nach der Implantierung der Leiding-Schweinezellen ist in der folgenden Tabelle angegeben.

| Testosteron -niveau | Die Dauer der Implantierung der Zellen im vorgeschlagenen Hydrogel | | | | | | |
|---|---|---|---|---|---|---|---|
| | 0 Mon. | 3 Mon. | 6 Mon. | 10 Mon. | 12 Mon. | 16 Mon. | 22 Mon. |
| Der 1. Patient 52 Jahre alt | 4,8 | 8,6 | 10,8 | 16,0 | 12,0 | 16,2 | 18,0 |
| | nmol/l | nmol/l | nmol/l | nmol/l | nmol/l | nmol/l | nmol/l |
| Der 2. Patient 38 Jahre alt | 6,8 | 12,6 | 16,4 | 18,6 | | 22,4 | |
| | nmol/l | nmol/l | nmol/l | nmol/l | | nmol/l | |
| Der 3. Patient 50 Jahre alt | 3,8 | 5,8 | 10,8 | 10,6 | 8,0 | 6,2 | 0 |
| | nmol/l | nmol/l | nmol/l | nmol/l | nmol/l | nmol/l | |
| Der 4. Patient 36 Jahre alt | 5,6 | 8,6 | 13,4 | 12,6 | 9,5 | 8,4 | 5,2 |
| | nmol/l | nmol/l | nmol/l | nmol/l | nmol/l | nmol/l | nmol/l |

Wie man aus dieser Tabelle zu ersehen ist, sichert das vorgeschlagene Hydrogel die Möglichkeit zur Kultivierung von Leiging-Schweinezellen im Organismus des Menschen unter Erhaltung der Fähigkeit dieser Zellen, das Testosteron im Laufe von 22 Monaten zu synthetisieren, während bei der Implantation der Zellen in den Gel-Prototyp (Formakril) die Fähigkeit zur aktiven Synthese des Hormons schon in 10 Monaten zu sinken beginnt

### Beispiel 4

Das vorgeschlagene Hydrogel wird zur Kultivierung von heterogenen Zellen der Bauchspeicheldrüse im Organismus des Menschen für die Behandlung von insulinabhängiger Diabetes verwendet.
1. Bei der Kranken F., 37 Jahre alt, wurde eine insulinabhängige Diabetes vor 11 Jahren, ein Jahr nach der Entbindung, diagnostiziert. Die Schwangerschaft der Kranken verlief schwer mit einer Toxikose in der zweiten Hälfte der Schwangerschaftszeit, mit einer Nephropathologie und mit einer wesentlichen Gewichtszunahme von 26kg. Die Krankheit hatte alle diese Jahre einen nicht stabilen Charakter, wodurch viel Mühe bei der Auswahl der adäquaten Insulintherapie entstand. Der Verbrauch des exogenen Insulins variierte von 58 Einheiten/Tag bis zu 30 Einheiten/Tag. In den letzten zwei Jahre sind pathologische Veränderungen der Nieren diagnostiziert worden, wobei diese Veränderungen als diabetische Nephropathie definiert werden konnten. In den Urin-Analysen war eine Steigerung der oberen Grenze der Proteinurie um das 10-12-Fache zu verzeichnen. Die Erhöhung des arteriellen Blutdrucks betrug bis zu 170/110mm Hg.
   Das wie im beschriebenen Beispiel gewonnene Hydrogel wurde dieser Kranken implantiert. Nach der Bildung der Bindegewebskapsel um das Gel wurde in das Gel eine Zellkultur der Bauchspeicheldrüse von neugeborenen Kaninchen eingebracht.
   Schon in 7 Tagen nach der Einbringung der Zellen der Bauchspeicheldrüse stellte die Patientin eine Verbesserung des Allgemeinzustands, eine Senkung des Durstgefühls und der Trockenheit der Mundschleimhaut und eine Senkung des arteriellen Blutdrucks bis zu 140/90mm Hg fest. In 15 Tagen erlaubte der Zustand der Patientin, den Bedarf an exogenem Insulin von 30 Einheiten bis zu 18 Einheiten herabzusetzen (Kontrolle des Bluts und des Urins). In 30 Tagen sank der Bedarf an exogenem Insulin bis 12 Einheiten/Tag und bis zum Ende des 2. Monats bis zu 4 Einheiten/Tag.
   Nach der Einführung der Zellen der Bauchspeicheldrüse wurde die Kranke im Laufe von 12 Monaten beobachtet. Klinische Erscheinungen der Nephropathie ließen sich nicht erkennen, der arterielle Blutdruck befand sich in den Grenzen der Altersnormen. Die Patientin wurde auf perorale Antidiabetika mit der obligatorischen Bedingung der Beachtung von Diabetes-Kost und der Kontrolle der Glykose im Blut, im Urin und des glykosylierten Homoglobins gesetzt.
2. Am Kranken K., 52 Jahre alt, wurde insulinabhängige Diabetes seit dem 18. Lebensjahr mit einer heftigen Stresssituation im Hintergrund diagnostiziert. Der Charakter der Erkrankung war am Anfang sehr unstabil. Die Dosen des exogenen Insulins erreichten 70 Einheiten/Tag.
   Mit der Zeit stabilisierte sich der Verlauf der Erkrankung, die Verschlechterungen des Zustands entstanden nach stressigen Situationen und Versäumnissen in der Diät. In den letzten drei Jahren war die Verschlechterung der Gefäße der unteren Gliedmaßen, die Senkung des Libidos und die Verschlechterung der Erektion und der Qualität des Geschlechtakts zu verzeichnen. Es wurde eine diabetische Angiopathie der unteren Gliedmaßen und des Penis diagnostiziert. Der Bedarf an exogenem Insulin betrug im letzten Jahr 20 Einheiten/Tag bis 40 Einheiten/Tag. Das nach dem beschriebenen Beispiel 1 gewonnene Hydrogel wurde diesem Kranken implantiert. Nach der Bildung der Bindegewebskapsel um das Gel wurde in das Gel die Zellkultur der Bauchspeicheldrüse von 14-tägigen Ferkeln eingebracht.
   In zwei Wochen nach der Einführung der Zellen der Bauchspeicheldrüse stellte der Patient eine Verbesserung des Allgemeinzustands fest. In einem Monat sank der Bedarf an exogenem Insulin bis zu 12 Einheiten/Tag und in zwei Monaten bis 6 Einheiten/Tag. In 4 Monaten nach der Transplantation wurde der Kranke auf perorale Antidiabetika gesetzt. Das sexuelle Leben des Kranken hat sich normalisiert, der Zustand der unteren Gliedmaßen hat sich wesentlich gebessert.

Die subjektiven und objektiven Symptome der untersuchten Patienten und die Angaben der zusätzlichen Forschungsmethoden (Blut, Urin) erlauben, über die hohe Effektivität der Behandlung der Zuckerkrankheit mittels Einführung von heterogenen Zellen der Bauchspeicheldrüse in das Hydrogel der vorgeschlagenen Zusammensetzung zu reden.

Der therapeutische Effekt dauert in der Regel 10 bis 20 Monate in Abhängigkeit von der Schwere der Erkrankung. Die Anzahl der zu transplantierenden Zellen wird auch durch die Schwere des Verlaufs der Zuckerkrankheit bestimmt, insbesondere durch die Menge des vom Kranken eingenommenen Insulins.

### Industrielle Anwendbarkeit

Die angeführten Ausführungsbeispiele bestätigen, dass das vorgeschlagene bioverträgliche Hydrogel mit dem vorgeschlagenen Verfahren gewonnen werden kann. Das vorgeschlagene Hydrogel ruft praktisch keine Gewebereaktion, keine Sensibilisation des Organismus sowie keine dystrophischen und keine nekrotischen Veränderungen hervor und kann für die Implantierung in den Organismus von Tieren und Menschen zur Bildung der Kapsel und zur weiteren Kultivierung der gewünschten Zellkulturen in der Kapsel eingesetzt werden. Im Vergleich zum bekannten Hydrogel-Prototyp (Hydrogel "Formakril") fehlen in der Struktur des vorgeschlagenen Hydrogels die freien NH₂-Radikale, welche die koordinative Bindung im Strukturnetz des Hydrogels bilden könnten. Das vorgeschlagene Hydrogel besitzt im Gegensatz zum Prototyp eine bessere Feinzellenstruktur und sichert die Möglichkeit einer längeren Kultivierung der implantierten Zellen im Organismus des Empfängers unter Erhaltung der Existenzfähigkeit der Zellen und der Fähigkeit, die für den Menschen erforderlichen Produkte, insbesodere Testosteron und Insulin, zu produzieren.

## Patentansprüche

1. Mehrfunktionelles, bioverträgliches Hydrogel, das Polyacrylamid und Wasser enthält,
**dadurch gekennzeichnet,**
**dass** das Polyacrylamid das Kopolymer von Acrylamid, Metacrylamid, 2-Hydroxyethyl-Methacrylat und das Vernetzungsmittel 2-Hydroxyethyl-Methacrylat und N,N'-Methylen-bis-Acrylamid in folgendem Verhältnis der Komponenten in Mass.% enthält:
- Acrylamid: 65,0-80,0,
- Metacrylamid: 18,0-30,0,
- 2-Hydroxyethyl-Metacrylat: 0,1-4,0
- N,N'-Methylen-bis-Acrylamid: 0,2-6,0.

2. Mehrfunktionelles, bioverträgliches Hydrogel nach Anspruch 1,
**dadurch gekennzeichnet,**
**dass** es das genannte Polyacrylamid mit 3,0 bis 10,0 Mass.% der Gesamtmasse des bioverträglichen Hydrogels enthält.

3. Mehrfunktionelles, bioverträgliches Hydrogel nach einem der Ansprüche 1 bis 2,
**dadurch gekennzeichnet,**
**dass** es folgendes Verhältnis der Komponenten in Mass.% enthält:
- Acrylamid: 1,95-8,0,
- Metacrylamid: 0,54-3,0,
- 2-Hydroxiäthyl-MetAcrylat: 0,003-0,4,
- N,N'-Methylen-bis-Acrylamid: 0,006-0,6,
- Wasser: Rest bis 100.

4. Mehrfunktionelles, bioverträgliches Hydrogel nach einem der Ansprüche 1 bis 3,
**dadurch gekennzeichnet,**
**dass** es als Wasser das bidestillierte Wasser Apyrogenwasser enthält.

5. Mehrfunktionelles, bioverträgliches Hydrogel nach einem der Ansprüche 1 bis 4,
**dadurch gekennzeichnet,**
**dass** es einen pH-Wert von 3,5-4,5 aufweist.

6. Mehrfunktionelles, bioverträgliches Hydrogel nach Anspruch 1,
**dadurch gekennzeichnet,**
**dass** es zum Injizieren geeignet ist.

7. Mehrfunktionelles, bioverträgliches Hydrogel nach Anspruch 1,
**dadurch gekennzeichnet,**
**dass** es zur Bildung einer Kapsel in den Geweben von Tieren und Menschen geeignet ist.

8. Mehrfunktionelles, bioverträgliches Hydrogel nach Anspruch 1,
**dadurch gekennzeichnet,**
**dass** es zur Kultivierung von heterogenen (allogenen und xenogenen) oder patienteneigenen Zellen geeignet ist.

9. Mehrfunktionelles, bioverträgliches Hydrogel nach Anspruch 6,
**dadurch gekennzeichnet,**
**dass** es in Spritzen abgefüllt ist.

10. Verfahren zur Gewinnung von mehrfunktionellem, bioverträglichen Hydrogel durch Kopolymerisation von Monomeren und Vernetzungsagenzien in wässrigem Medium in Anwesenheit eines Peroxidinitiators der Polymesierung in mehreren Stufen,
**dadurch gekennzeichnet,**
**dass** als Monomere Acrylomid und Methacrylamid und als Vernetzungsagenzien Hydroxiäthyl-Metacrylat und N,N'-Methylen-bis-Acrylamid bei folgendem Verhältnis der Komponenten in Mass.% genommen werden:
- Acrylamid: 1,95-8,0,
- Methacrylamid: 0,54-3,0,
- 2-Hydroxiäthyl-Metacrylat: 0,003-0,4,
- N,N'-Methylen-bis-Acrylamid: 0,006-0,6,
- Wasser: Rest bis 100,
wobei die Kopolymerisation in drei Stufen durchgeführt wird, nämlich die erste Stufe bei einer Zimmertemperatur von 20-30 °C innerhalb von 12-24 Stunden, die zweite Stufe durch γ-Bestrahlung in einer Dosis von 0,4-1,0 Megarad und die dritte Stufe bei einer Temperatur von 100-130 °C und einem Druck von 0-1,2 Atmosphären innerhalb von 20-40 Minuten.

11. Verfahren nach Anspruch 10,
**dadurch gekennzeichnet,**
**dass** das genannte Hydrogel nach der ersten Stufe der Kopolymerisation mit heißem Wasser, das eine Temperatur von 70-110 °C aufweist, mindestens 3 Stunden lang bei einem Druck von 0-1,2 Atmosphären und bei einem Massenverhältnis des Hydrogels zum Wasser von 1:8-10 abgespült wird.

12. Verfahren nach Anspruch 10,
**dadurch gekennzeichnet,**
**dass** als Kopolymerisationsinitiator Wasserstoffperoxid und/oder Peroxodisulfat-Ammonium in einer Menge von nicht mehr als 0,33 Mass.% des summarischen Gewichts der Ausgangskomponenten genommen wird bzw. werden.

13. Verfahren nach Anspruch 10,
**dadurch gekennzeichnet,**
**dass** als Wassermedium bidestilliertes Apyrogenwasser genommen wird.

14. Verfahren nach Anspruch 10,
**dadurch gekennzeichnet,**
**dass** das genannte Hydrogel in Spritzen abgefüllt wird.

## Claims

1. Multifunctional, biocompatible hydrogel that contains polyacrylamide and water, **characterised in that** the polyacrylamide contains the copolymer of acrylamide, methacrylamide, 2-hydroxyethyl methacrylate and the cross-linking agent 2-hydroxyethyl methacrylate and N,N'-methylene-bis-acrylamide, the components being in the following proportions with % by weight:
- acrylamide: 65,0-80.0,
- methacrylamide: 18.0- 30.0,
- 2-hydroxyethyl methacrylate: 0.1-4.0,
- N, N'-methylene-bis-acrylamide: 0.2-6.0.

2. Multifunctional, biocompatible hydrogel according to claim 1, **characterised in that** the said hydrogel contains the abovementioned polyacrylamide at between 3.0 and 10.0 percent by weight of the total weight of the bio compatible hydrogel.

3. Multifunctional, biocompatible hydrogel according to one of claims 1 to 2, **characterised in that** the said hydrogel contains components in the following proportions (percent by weight):
- acrylamide: 1.95 - 8.0,
- methacrylamide: 0.54 - 3.0,
- 2-hydroxyethyl methacrylate: 0.003 - 0.4,
- N,N'-methylene-bis-acrylamide: 0.006 - 0.6,
- Water: remainder to 100.

4. Multifunctional, biocompatible hydrogel according to one of claims 1 to 3, **characterised in that** the said hydrogel contains the bidistilled water - apyrogenic water - as water.

5. Multifunctional, biocompatible hydrogel according to one of claims 1 to 4, **characterised in that** the said hydrogel has a pH value of between 3.5 and 4.5.

6. Multifunctional, biocompatible hydrogel according to claim 1, **characterised in that** the said hydrogel is suitable for injection.

7. Multifunctional, biocompatible hydrogel according to claim 1, **characterised in that** the said hydrogel is suitable for forming a capsule in human and animal tissues.

8. Multifunctional, biocompatible hydrogel according to claim 1, **characterised in that** the said hydrogel is suitable for cultivating heterogeneous (allogenic and xenogenic) or autoimmune cells.

9. Multifunctional, biocompatible hydrogel according to claim 6, **characterised in that** the said hydrogel is packed in syringes.

10. Method for obtaining multifunctional, biocompatible hydrogel by means of the several stage copolymerisation of monomers and cross-linking agents in an aqueous medium in the presence of a peroxide initiator of the polymerisation, **characterised in that** acrylamide and methacrylamide are used as monomers and hydroxyethyl methacrylate and N,N'-methylene-bis-acrylamide are used as cross-linking agents with the components in the following proportions with % by weight:
- acrylamide: 1.95 - 8.0,
- methacrylamide: 0.54 - 3.0,
- 2-hydroxyethyl-methacrylate: 0.003 - 0.4,
- N, N'-methylene-bis-acrylamide: 0.006 - 0.6,
- water: remainder to 100,
wherein the copolymerisation is carried out in three stages, namely the first stage at a room temperature of between 20 and 30°C for 12 to 24 hours, the second stage by means of exposure to y radiation at a dose of between 0.4 and 1.0 megarads and the third stage at a temperature of between 100 and 130°C and at a pressure of between 0 and 1.2 atm. for 20 to 40 minutes.

11. Method according to claim 10, **characterised in that** after the first stage of the copolymerisation, the abovementioned hydrogel is washed with hot water, which is at a temperature of between 70 and 110°C, for at least 3 hours at a pressure of between 0 and 1.2 atm. and at a ratio of the weight of the hydrogel to the water of 1: 8 - 10.

12. Method according to claim 10, **characterised in that** hydrogen peroxide and/or peroxo bisulphate ammonium is or respectively are used as the copolymerisation initiator at an amount of no more than 0.33 % by weight of the total weight of the initial components.

13. Method according to claim 10, **characterised in that** bidistilled apyrogenic water is used as the water medium.

14. Method according to claim 10, **characterised in that** the abovementioned hydrogel is packed in syringes.

## Revendications

1. Hydrogel biocompatible et multifonctionnel, qui comprend du polyacrylamide et de l'eau,
**caractérisé,**
**en ce que** le polyacrylamide comprend le copolymère d'acrylamide, de méthacrylamide, de méthacrylate d'hydroxyéthyle et l'agent de réticulation comprend du méthacrylate de 2- hydroxyéthyle et du N,N' méthylène bis acrylamide en le rapport suivant des constituants en pourcentages en masse :
- acrylamide :65,0 à 80,0,
- méthacrylamide : 18,0 à 30,0,
- méthacrylate de 2- hydroxyéthyle : 0,1 à 4,0
- N,N'méthylène bis acrylamide : 0,2 à 6,0.

2. Hydrogel biocompatible et multifonctionnel suivant la revendication 1,
**caractérisé**
**en ce qu'**il contient le polyacrylamide mentionné ci-dessus représentant de 3,0 à 10,0 % de la masse totale de l'hydrogel biocompatible.

3. Hydrogel biocompatible et multifonctionnel suivant la revendication 1 à 2, **caractérisé en ce qu'**il contient les constituants en leur rapport suivant en pourcentages en masse,
- acrylamide :1,95 à 8,0,
- méthacrylamide: 0,54 à 3,0,
- méthacrylate de 2- hydroxyéthyle : 0,003 à 0,4,
- N,N'méthylène bis acrylamide : 0,006 à 0,6,
- eau : le reste jusqu'à 100.

4. Hydrogel biocompatible et multifonctionnel suivant l'une des revendications 1 à 3,
**caractérisé en ce qu'**il contient comme eau de l'eau apyrogène bidistillée.

5. Hydrogel biocompatible et multifonctionnel suivant l'une des revendications 1 à 4,
**caractérisé en ce qu'**il a un pH de 3,5 à 4,5.

6. Hydrogel biocompatible et multifonctionnel suivant la revendication 1,
**caractérisé en ce qu'**il est approprié pour l'injection.

7. Hydrogel biocompatible et multifonctionnel suivant la revendication 1, **caractérisé en ce qu'**il est approprié pour former une capsule dans les tissus de l'animal et de l'homme.

8. Hydrogel biocompatible et multifonctionnel suivant la revendication 1, **caractérisé en ce qu'**il est approprié pour la culture de cellules hétérogènes (allogènes et xénogénènes) ou propres au patient.

9. Hydrogel biocompatible et multifonctionnel suivant la revendication 6, **caractérisé en ce qu'**il est transvasé dans des seringues.

10. Procédé d'obtention d'hydrogel biocompatible et multifonctionnel, par copolymérisation de monomères et d'agents de réticulation dans un milieu aqueux en la présence d'un peroxyde amorceur de la polymérisation en plusieurs stades,
**caractérisé**
**en ce que** l'on prend comme monomères de l'acrylamide et du méthacrylamide et comme agents de réticulation du méthacrylate d'hydroxyéthyle et du N, N' méthylène bis acrylamide en le rapport suivant des constituants en pourcentages en masse :
- acrylamide :1,95 à 8,0,
- méthacrylamide : 0,54 à 3,0,
- méthacrylate de 2 hydroxyéthyle : 0,003 à 0,4,
- N,N'méthylène bis acrylamide : 0,006 à 0,6,
- eau : reste jusqu'à 100,
en effectuant la copolymérisation en trois stades, à savoir le premier stade à une température ambiante de 20 à 30°C en 12 à 24 heures, le deuxième stade par exposition du rayonnement gamma en une dose de 4,4 à 1,0 mégarad et le troisième stade à une température de 100 à 130°C sous une pression de 0 à 1,2 atmosphère en 20 à 40 minutes.

11. Procédé suivant la revendication 10,
**caractérisé**
**en ce que** l'on lave ledit hydrogel après le premier stade de la copolymérisation à l'eau chaude ayant une température de 70 à 110°C pendant au moins au moins 3 heures sous une pression de 0 à 1,2 atmosphère et avec un rapport en masse de l'hydrogel à l'eau de 1:8 à 10.

12. Procédé suivant la revendication 10,
**caractérisé**
**en ce que** l'on prend comme amorceur de copolymérisation du peroxyde d'hydrogène et/ou du peroxodisulfate d'ammonium en une quantité ne représentant pas plus de 0,33 % en masse de la somme des poids des constituants de départ.

13. Procédé suivant la revendication 10,
**caractérisé**
**en ce que** l'on prend comme milieu aqueux de l'eau apyrogène bidistillée.

14. Procédé suivant la revendication 10,
**caractérisé**
**en ce que** ledit hydrogel est transvasé dans des seringues.
